(19) Europäisches Patentamt
European Patent Office
Office européen des brevets

(11) **EP 2 752 177 B1**

(12) **EUROPEAN PATENT SPECIFICATION**

(45) Date of publication and mention
of the grant of the patent:
**07.03.2018 Bulletin 2018/10**

(21) Application number: **12828584.8**

(22) Date of filing: **23.08.2012**

(51) Int Cl.:
*A61F 13/15* *(2006.01)*       *A61F 13/494* *(2006.01)*
*A61F 13/49* *(2006.01)*       *A61F 13/56* *(2006.01)*

(86) International application number:
**PCT/JP2012/071336**

(87) International publication number:
**WO 2013/031645 (07.03.2013 Gazette 2013/10)**

(54) **DISPOSABLE DIAPER**

WEGWERFWINDEL

COUCHE JETABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.08.2011   JP 2011189101**

(43) Date of publication of application:
**09.07.2014   Bulletin 2014/28**

(73) Proprietor: **Unicharm Corporation
Ehime 799-0111 (JP)**

(72) Inventors:
• **OKU, Tomomi
Kanonji-shi
Kagawa 769-1602 (JP)**

• **MATSUSHIMA, Hideki
Kanonji-shi
Kagawa 769-1602 (JP)**

(74) Representative: **Dolleymores
9 Rickmansworth Road
Watford, Hertfordshire WD18 0JU (GB)**

(56) References cited:
**WO-A1-2011/040045       WO-A1-2011/040046
JP-A- H07 452             JP-A- 2010 233 927
JP-A- 2011 072 679        JP-A- 2011 078 647**

**Description**

[Technical Field]

The present invention relates to a disposable diaper including a diaper main body and a side flap, in which the diaper main body has an absorber provided between a topsheet and a backsheet, and the side flap projects outwardly beyond an outer side in a widthwise direction from the diaper main body.

[Background Art]

Conventionally, an open-type disposable diaper has been provided as an absorbent article (for example, see Patent Literature 1). The open-type disposable diaper includes a diaper main body having an absorber provided between a topsheet and a backsheet, and a dorsal side flap projecting outwardly beyond an outer side in a widthwise direction from the diaper main body, at an outer end in a longitudinal direction of the diaper main body. This open-type disposable diaper is provided in a state in which each member is spread out.

The dorsal side flap of the disposable diaper described in Patent Literature 1 is provided with a fixing member configured to fix the dorsal side flap to the diaper main body in an attachable and detachable manner. At both outer ends of the diaper main body in a widthwise direction, elastic members configuring leg gathers are configured so as to be stretchable in the longitudinal direction.

When wearing the disposable diaper, at first the absorber is arranged facing the crotch of a wearer while the dorsal side flap is positioned at the dorsal side of the wearer. Thereafter, the dorsal side flap is pulled towards a ventral side of the wearer and the fixing member is fixed to the diaper main body at the ventral side of the wearer. Accordingly, a waistline of the wearer is covered with the dorsal side flap and the diaper main body while legs of the wearer are covered with the outer ends of the diaper main body in the widthwise direction. Further, at the outer ends of the diaper in the widthwise direction, the elastic members configuring the leg gathers are arranged so that the diaper main body is fitted onto the legs of the wearer.

[Citation List]

[Patent Literature]

[PTL 1] Japanese Unexamined Patent Publication No. 2000-513636 (Fig. 1, etc.)

[Summary of Invention]

However, the Applicant found the following problem associated with the abovementioned disposable diaper.

At an approximately center in the longitudinal direction and the widthwise direction of the disposable diaper, a crotch region corresponding to a crotch portion of the wearer is provided. This crotch region is separated from the dorsal side flap corresponding to the waistline of the wearer. Therefore, when the absorber gets heavier after bodily fluid or the like is discharged, the crotch region sags down due to weight of the bodily fluid or the like, thereby possibly being separated from the wearer.

At this time, the dorsal side flap, which is positioned at both outer ends in the longitudinal direction of the disposable diaper, and the diaper main body are joined together by a fixing member, so that even in a case where the crotch region sags down due to weight of the absorber, a state is easily maintained in which the dorsal side flap is arranged onto the waistline of the wearer.

However, the elastic member arranged in each leg of the wearer is separated from the side flap provided with the fixing member, thereby possibly sagging down in association with the sagging down of the crotch region. When the elastic member sags down, the leg gather cannot be formed so as to run around each of the legs of the wearer, thereby leading to the possibility of deterioration in fitting ability at the legs of the wearer. When the fitting ability at the legs is deteriorated, a gap is formed between the legs of the wearer and the disposable diaper, thereby leading to the possibility of causing side leakage.

Therefore, an object of the present invention is to provide a disposable diaper which is capable of improving the fitting ability at the legs of the wearer to thereby control occurrence of side leakage by arranging elastic members configuring leg gathers which run around the legs of a wearer.

The present invention provides the disposable diaper of independent claim 1. The dependent claim specifies preferred but optional features. WO 2011/040046 and WO 2011/040045 disclose a diaper having a similar configuration to that presently claimed. In those diapers the outermost elastic member overlaps the non-joining unit. A disposable diaper according to the present invention includes a diaper main body (diaper main body 10) having: a liquid-permeable topsheet (topsheet 110); a liquid-impermeable backsheet (backsheet 120); an absorber (absorber 130) provided between the topsheet and the backsheet; and a plurality of elastic members (first elastic member 123a,123b,124a,124b, second elastic member 123c,124c) arranged in an extended state along a longitudinal direction of the absorber, at outer sides in a widthwise direction with respect to the absorber; side flaps (dorsal side flap 20, ventral side flap 30) projecting outwardly beyond outer sides in the widthwise direction with respect to the diaper main body, at an outer end of the diaper main body in the longitudinal direction; and a joining unit (joining portion 51,53) configured to join the side flap and the diaper main body, wherein an elastic member (first elastic member 123a,124a) positioned at outermost sides in the widthwise direction out of the plurality of elastic members is overlapped in an extended state in the longitudinal direction, with the joining unit in a thickness direction (thickness direction T) of the disposable diaper.

[Brief Description of Drawings]

[Fig. 1] Fig. 1 is a plan view of a disposable diaper according to a first embodiment.
[Fig. 2] Fig. 2 is an assembly drawing of the disposable diaper according to a first embodiment.
[Fig. 3] Fig. 3 is a cross-sectional view taken along the line A-A' shown in Fig. 1.
[Fig. 4] Fig. 4 is a cross-sectional view taken along the line B-B' shown in Fig. 1.
[Fig. 5] Fig. 5 is a cross-sectional view taken along the line C-C' shown in Fig. 1.
[Fig. 6] Fig. 6 is a perspective view for schematically showing a spread-out state of the disposable diaper.
[Fig. 7] Fig. 7 is a view for schematically showing a wearing state of the disposable diaper 1
[Fig. 8] Fig. 8 is a view for illustrating a positional relationship between a fixing member of a side flap and an elastic member.
[Fig. 9] Fig. 9 is an assembly drawing for illustrating a disposable diaper according to a second embodiment.

[Description of Embodiment]

(Disposable Diaper According to First Embodiment)

A disposable diaper according to a first embodiment is explained with reference to the drawings. Note that in the description of the following drawings, the same or similar symbols are added to the same or similar portions. However, it must be taken into consideration that the drawings are schematic representations and are not drawn to scale unless otherwise specified. Moreover, the drawings do not necessarily reflect the actual dimensional relationships and ratios of components

A specific dimension should be determined in view of the following description. Further, among the drawings, the respective dimensional relations or ratios may naturally differ. Yet further, the same reference numerals have been used for configurations having the same operation and effect while omitting the detailed explanation.

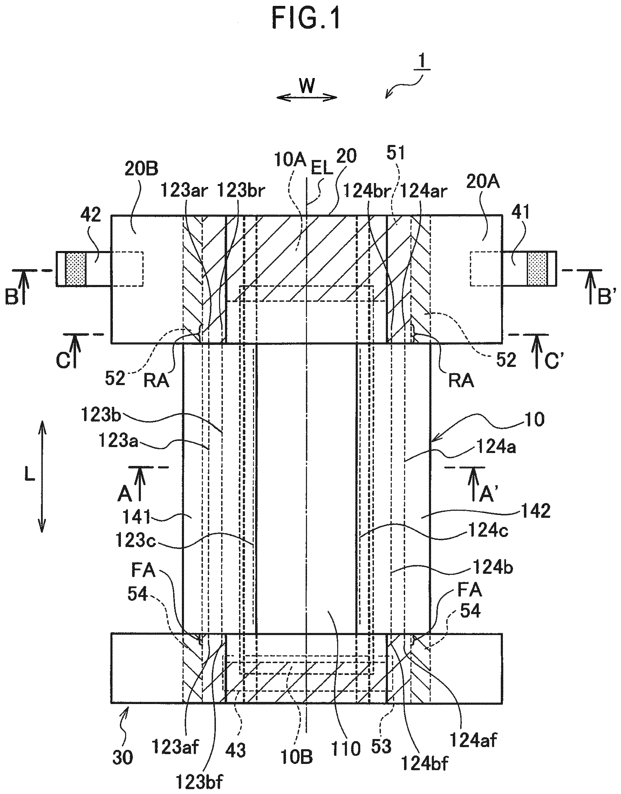

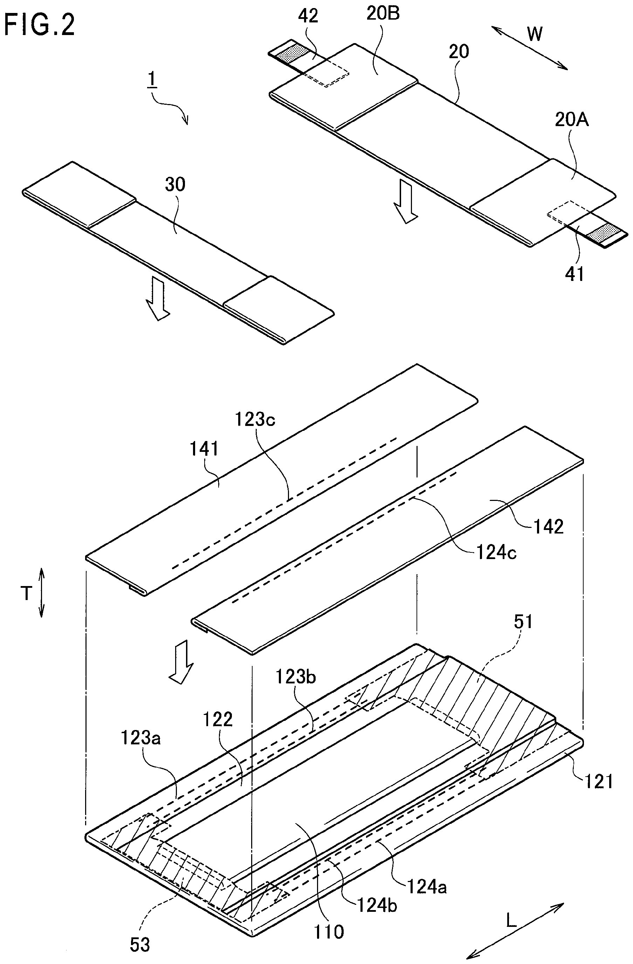

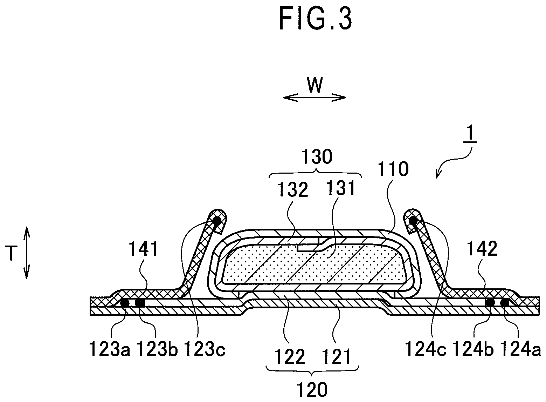

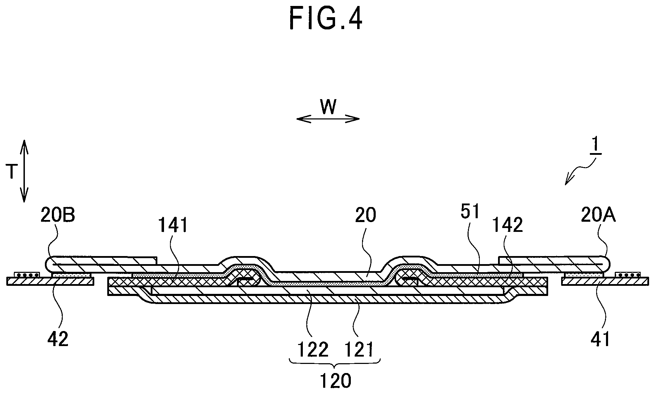

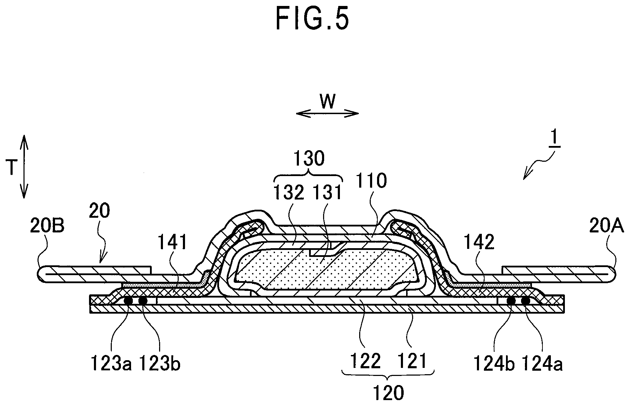

Fig. 1 is a plan view of a disposable diaper 1 according to the first embodiment. Fig. 2 is an assembly drawing of the disposable diaper 1 shown in Fig. 1. Fig. 3 is a cross-sectional view of the disposable diaper 1 taken along the line A-A'. Fig. 4 is a cross-sectional view of the disposable diaper 1 taken along the line B-B'. Fig. 5 is a cross-sectional view of the disposable diaper 1 taken along the line C-C'.

The disposable diaper 1 includes a diaper main body 10, a dorsal side flap 20, and a ventral side flap 30. The dorsal side flap 20 of the disposable diaper 1 is placed at a dorsal side of a waistline of a wearer. The ventral side flap 30 is placed at a ventral side of the waistline of the wearer. The diaper main body 10 is placed at the crotch of the wearer.

The diaper main body 10 has a rectangular shape. The diaper main body 10 includes: a liquid-permeable topsheet 110 which is arranged on a wearer's side; a liquid-impermeable exterior sheet 120 as a backsheet, which is arranged on the clothing side; an absorber 130 which is arranged between the topsheet 110 and the exterior sheet 120; and sidesheets 141, 142 which are provided at outer sides in a widthwise direction of the absorber 130.

The topsheet 110 is not particularly limited as long as the topsheet 110 is a sheet-like material having a structure that allows liquids to pass through, such as a nonwoven fabric, a woven fabric, a perforated plastic sheet, and a mesh sheet. As a material for a woven fabric and a nonwoven fabric, both of a natural fiber and a chemical fiber can be used.

Examples of the natural fiber include cellulose such as ground pulp and cotton. Examples of the chemical fiber include regenerated cellulose such as rayon and fibril rayon, semisynthetic cellulose such as acetate and triacetate, thermoplastic hydrophobic chemical fibers, and thermoplastic hydrophobic chemical fibers subjected to hydrophilization treatment. Examples of the thermoplastic hydrophobic chemical fiber include a single fiber such as polyethylene (PE), polypropylene (PP), and polyethylene terephthalate (PET), a fiber obtained by graft polymerization of polyethylene and polypropylene, and a composite fiber with a sheath-core structure and the like. For example, the topsheet 110 is point bond nonwoven fabric having a basis weight of 23 g/m$^2$.

As a method of forming nonwoven fabric, any one of dry methods (the carding method, the spun bonding method, the melt-blowing method, the air-laid method, etc.) and wet methods may be used. Alternatively, out of the dry methods and wet methods, a plurality of methods may be used in combination. Moreover, thermal bonding, needle punching, and chemical bonding may be included. However, the method of forming nonwoven fabric is not particularly limited to the above methods.

The exterior sheet 120 has a back nonwoven fabric 121 which comes in contact with the clothing, and a liquid-impermeable film (hereinafter, referred to as a back film) 122 which is positioned closer to a skin side than the back nonwoven fabric 121. The back film 122 is made from a moisture-permeable or moisture-impermeable film. The back nonwoven fabric 121 is a hydrophobic nonwoven cloth configured from an SMS nonwoven cloth (a composite nonwoven cloth of a spun bond nonwoven cloth and a melt-blown nonwoven cloth), a spun bond nonwoven cloth, or a point bond nonwoven cloth. The back nonwoven fabric 121 and the back film 122 are joined together by means of a hot-melt adhesive (hereinafter, referred to as HMA) or the like. For example, the back film 122 is a non-breathable film having a basis weight of 16 g/ m$^2$, and the back nonwoven fabric 121 is an SMS nonwoven cloth having a basis weight of 13 g/m$^2$.

The absorber 130 has an absorbent core 131 configured to absorb liquids, and an absorbent sheet 132. The absorbent core 131 includes hydrophilic fibers and highly absorbent polymer. Examples of the hydrophilic fiber include cellulose such as ground pulp and cotton, regenerated cellulose such as rayon and fibril rayon, semisynthetic cellulose such as acetate and triacetate, granular polymers, fibrous polymers, thermoplastic hydrophobic chemical fibers, and thermoplastic hydrophobic chemical fibers subjected to hydrophilization treatment. These can be used alone or in combination. Out of these, in consideration of lower costs and workability of forming the absorber, it is desirable that ground pulp be used. A mixture obtained by mixing the highly absorbent polymer with the hydrophilic fibers may be used.

The absorbent core 131 is wrapped with the absorbent sheet 132. In the present embodiment, the absorbent sheet 132 is a tissue. The absorber 130 is joined to a surface at the skin side of the back film 122 by means of an HMA or the like in a state that the absorber 130 is wrapped with the topsheet 110.

The sidesheets 141, 142 are arranged at both sides of the topsheet 110 in the widthwise direction. The sidesheets 141, 142 are joined to the back nonwoven fabric 121 at the outer sides in the widthwise direction with respect to the back film 122. The first elastic members, which are to be described later, are arranged between the sidesheets 141, 142 and the back nonwoven fabric 121.

The sidesheets 141, 142 may be selected from similar materials to that of the exterior sheet 120. However, in order to prevent excretions such as urine and stool from flowing into the outer sides of the disposable absorber 1 in the widthwise direction by crossing over the sidesheets 141, 142, it is desirable that the material have a hydrophobic property or water repellency.

A dorsal side flap 20 and a ventral side flap 30 are joined to both ends of the absorbent main body 10 in the longitudinal direction, respectively. The dorsal side flap 20 is mounted on at least one end region 10A of the absorbent main body 10 in the longitudinal direction. A width of the dorsal side flap 20 is longer than a width of the absorbent main body 10 and covers the end region 10A.

The ventral side flap 30 is mounted on the other end region 10B of the absorbent main body 10 in the longitudinal direction. A width of the ventral side flap 30 is longer than a width of the absorbent main body 10 and covers the end region 10B.

The dorsal side flap 20 and the ventral side flap 30 are made from a hydrophobic nonwoven cloth, a moisture-permeable or moisture-impermeable film, or a composite sheet formed by pasting together a hydrophobic nonwoven cloth and a moisture-permeable or moisture-impermeable film. A film with the main constituent as polyethylene or polypropylene, a breathable resin film, or a sheet in which a breathable resin film is joined to a nonwoven cloth such as spun bond or spun lace can be used.

The dorsal side flap 20 and the ventral side flap 30 according to the present embodiment are made from an SMS nonwoven cloth with a basis weight of 15 g/ m$^2$. At least at free ends 20A, 20B of the dorsal side flap 20 in the widthwise direction, the outer ends in the widthwise direction are folded over towards the inner side in the widthwise direction based on the outer ends in the widthwise direction, thereby being folded to form two layers.

Fixing members 41, 42 are provided at the dorsal side flap 20. The fixing members 41, 42 are provided at free ends 20A, 20B positioned at both ends of the dorsal side flap 20 in the widthwise direction, respectively.

A clothing contact surface side of an end region 10B of the diaper main body 10 is provided with a locking unit 43 into which the fixing members 41, 42 are locked. Each of the fixing members 41, 42 is a male member of a so-called hook-and-loop fastener while the locking unit 43 is a female member.

In the fixing members 41, 42, an engagement face having a group of plural hook-like projections (referred to as a hook) is formed. Examples of a material for the fixing members include a PPSB (polypropylene spunbonded nonwoven fabric), and for example, it is desirable to use PPSB having a basis weight of 80 g/ m$^2$. Abase weight of the hook is desired to be 100 g/ m$^2$.

The locking unit 43 into which the fixing member 40 is locked is a nonwoven cloth or knitted material into which the hook can be engaged. As a fiber material configuring the nonwoven cloth, a composite fiber having a core-clad structure is used. This fiber material adopts as a combination of a core component and a clad component, PP (polypropylene)/PE (polyethylene), PP/low-melting-point PP, PET (polyethylene terephthalate)/low-melting-point PET, and PET/PE. A fiber may be mixed with the fiber material, in which examples of the fiber include polyamide such as rayon, PET, PP, and nylon, acrylic, urethane, and cotton. For example, a basis weight of the female member is equal to 38 g/$m^2$.

The diaper main body 10 and the dorsal side flap 20 are joined to each other at a joining unit 51, by an adhesive such as HMA. Fig. 1 shows the joining unit 51 with diagonal lines. A length of the joining unit 51 in the longitudinal direction at the outer side in the widthwise direction is longer than a length of the joining unit 51 in the longitudinal direction at the central portion in the widthwise direction. The joining unit 51 is shaped so that the central portion in the widthwise direction is concave towards the outer side in the widthwise direction.

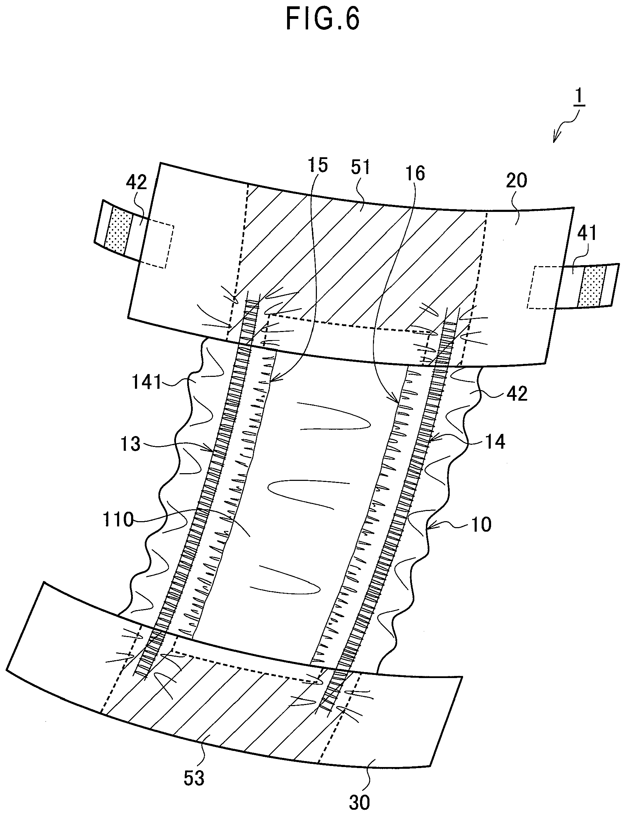

In the diaper main body 10, a plurality of elastic members is arranged in an extended state along a longitudinal direction L of the absorber 130. The elastic member includes first elastic members 123a, 123b, 124a, 124b configuring leg gathers 13 (see, Fig. 6) formed around the legs of the wearer, and second elastic members 123c, 124c configuring leakage-preventing walls 15 (see, Fig. 6) arranged at the crotch portion of the wearer. Note that the second elastic members are arranged at the inner ends of the sidesheets 141, 142 in the widthwise direction, and function as a side elastic member.

The plurality of elastic members are symmetrically arranged with respect to a virtual line EL as the central axis, which passes through the center of the disposable diaper in the widthwise direction while extending in the longitudinal direction. With the virtual line EL being as the central axis, the first elastic members 123a, 123b and the second elastic member 123c are arranged on one side while the first elastic members 124a, 124b and the second elastic member 124c are arranged on the other side.

The first elastic members 123a, 123b (the first elastic members 124a, 124b) are adjacently arranged in the widthwise direction. Further, the first elastic members 123a, 124a, which are arranged at the outer sides in the widthwise direction, are arranged at the outermost sides in the widthwise direction, among the plurality of elastic members.

A dorsal end region RA including dorsal ends 123ar, 123br, 124ar, 124br of the first elastic members 123a, 123b, 124a, 124b, is overlapped with respect to the joining unit 51 in a thickness direction T of the disposable diaper (as seen in a plan view shown in Fig. 1).

Note that a state in which the first elastic members 123a, 123b, 124a, 124b and the joining unit 51 are overlapped with each other in the thickness direction includes not only a state in which the first elastic members 123a, 123b, 124a, 124b and the joining unit 51 are overlapped in a contact state but also a state in which the first elastic members 123a, 123b, 124a, 124b and the joining unit 51 are overlapped while another member is sandwiched therebetween.

In the dorsal end region RA, the first elastic members 123a, 123b, 124a, 124b are arranged in an extended state along a longitudinal direction at the diaper main body 10. Therefore, elastic force of the first elastic members 123a, 123b, 124a, 124b is exerted in a direction of contracting the diaper main body 10 and the dorsal side flap 20 in the longitudinal direction L.

The diaper main body 10 and the ventral side flap 30 are joined to each other at a joining unit 53, by means of an adhesive such as HMA. Similarly, a ventral end region FA including ventral ends 123af, 123bf, 124af, 124bf of the first elastic members 123a, 123b, 124a, 124b is overlapped with the joining unit 53 in the thickness direction T of the disposable diaper (as seen in a plan view shown in Fig. 1).

In the ventral end region FA, the first elastic members 123a, 123b, 124a, 124b are arranged in an extended state along a longitudinal direction at the diaper main body 10. Therefore, elastic force of the first elastic members 123a, 123b, 124a, 124b is exerted in a direction of contracting the diaper main body 10 and the ventral side flap 30 in the longitudinal direction L.

The first elastic members 123a, 123b, 124a, 124b are joined by an adhesive such as HMA in an extended state in the longitudinal direction L, between a skin contact surface side of the back nonwoven fabric 121 at the outer sides in the widthwise direction with respect to the back film 122, and non-skin contact surface sides of the sidesheets 141, 142.

In the joining unit 51, the dorsal side flap 20, the sidesheets 141, 142, and the back nonwoven fabric 121 are contracted in the longitudinal direction L by means of the first elastic members. In the joining unit 53, the ventral side flap 30, the sidesheets 141, 142, and the back nonwoven fabric 121 are contracted in the longitudinal direction L by means of the first elastic members. The sidesheets 141, 142 and the back nonwoven fabric 121 are contracted in the longitudinal direction L between the joining portion 51 and the joining portion 53.

The second elastic members 123c, 124c are arranged at the inner sides in the longitudinal direction with respect to the dorsal side flap 20 and the ventral side flap 30, and configure the leakage-preventing walls 15, 16.

The second elastic members 123c, 124c are arranged on the sidesheets 141, 142, in an extended state along a longitudinal direction. The sidesheets 141, 142 are folded over towards the inner side so as to involve the second elastic members at the inner ends in the widthwise direction. By formation of the leakage-preventing walls 15, 16, excretions discharged from the wearer can be prevented from leaking from the sidesheets 141, 142 to the outer sides in the widthwise direction.

Both ends of each sidesheet in the longitudinal direction are arranged between the side flaps and the topsheet, and are joined to the side flaps and the topsheet, respectively, in a region which is overlapped with the absorber in the thickness direction. Even in a case where the absorber gets heavier after discharge of the bodily fluid or the like, the absorber can be pulled up through the sidesheets by stretching force exerted by the second elastic members. Therefore, the crotch region of the disposable diaper hardly sags down, thereby making it difficult to have a gap between the disposable worn article and the legs of the wearer.

Each of the first elastic members 123a, 123b, 124a, 124b according to the present embodiment is rubber having a diameter of 620 dtex. This rubber is arranged while it is stretched out 2.2 times. Further, each of the second elastic members 123c, 124c according to the present embodiment is rubber having a diameter of 620 dtex. This rubber is arranged while it is stretched out 2.5 times.

The joining units 51, 53 are provided up to a position which is 3 mm longer towards the outer side in the widthwise direction than the elastic member (the first elastic members 123a, 124a) positioned at the outermost side in the widthwise direction. By formation of the joining units 51, 53 in this manner, the elastic members can surely be arranged by being overlapped with the joining units 51, 53 even in the event of displacement at the time of transfer of the dorsal side flap 20 and the ventral side flap 30 to the diaper main body 10.

Further, a third elastic member (not shown) which is stretchable in the longitudinal direction may be provided at the inner side in the widthwise direction with respect to the second elastic members 123c, 124c configuring the leakage-preventing walls as well as at the outer side in the widthwise direction with respect to the absorber 130. For example, the third elastic member may be provided in an extended state in the longitudinal direction, between the back nonwoven fabric 121 and the back film 122. By arrangement of the third elastic member, the absorber can be prevented from sagging down from the crotch at the time of discharge of the bodily fluid, for example, at the time of urination, thereby achieving a higher effect in terms of fitting ability at the legs of the wearer.

Note that a stretch range of the third elastic member in the longitudinal direction is desired to be shorter than that of the first elastic member and not to reach the dorsal side flap 20. For example, if a stretch region of the third elastic member extends up to the dorsal side flap 20, the absorber itself is greatly contracted, thereby possibly causing difficulty in wearing the disposable diaper. Further, there is also a possibility that the contracted absorber comes in contact with the crotch portion of the wearer and the feeling of comfort at the time of wearing is decreased due to stiffness of the absorber.

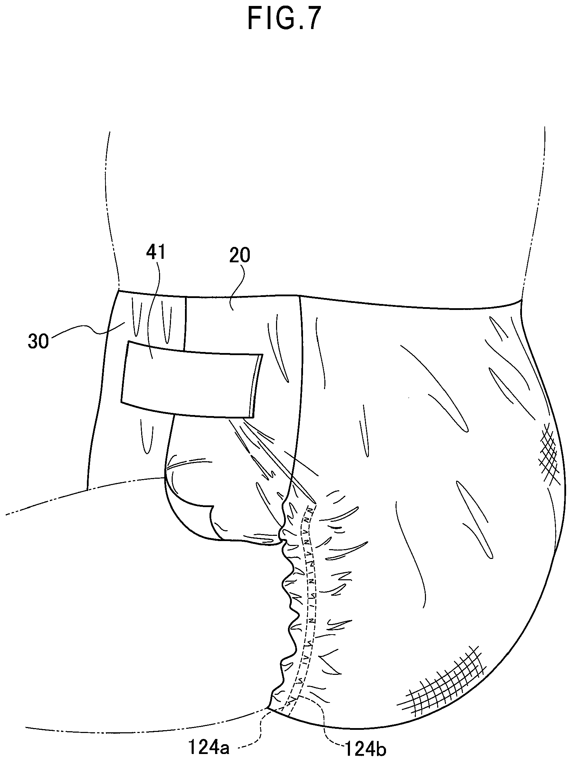

Fig. 6 is a view for schematically showing a spread-out state of the disposable diaper 1. Note that the spread-out state means a state in which the dorsal side flap 20 and the ventral side flap 30 are not joined together, as shown in Figs. 1 and 6. Fig. 7 is a view for schematically showing a state in which the disposable diaper is provided on the wearer.

As shown in Fig. 6, on the dorsal side flap 20 and the ventral side flap 30, the first elastic members 123a, 123b, 124a, 124b are arranged in a stretched-out state in the longitudinal direction L. Therefore, the dorsal side flap 20 and the ventral side flap 30 are configured so as to be stretchable in a spread-out state, in the longitudinal direction L by the first elastic members 123a, 123b, 124a, 124b.

In a case of using the disposable diaper 1 thus configured, as shown in Fig. 7, in a state in which the absorber 130 of the diaper main body 10 is brought in contact with the crotch of the wearer while the ventral side flap 30 is brought in contact with the belly of the wear, the fixing members 41, 42 arranged on both sides of the dorsal side flap 20 in the widthwise direction are fixed to the locking unit 43 of the ventral side flap.

In a state in which the fixing members 41, 42 are fixed to the locking unit 43 of the ventral side flap, the waistline of the wearer is covered with the dorsal side flap 20 and the ventral side flap 30. The dorsal side flap 20 and the ventral side flap 30 are joined together by means of the fixing member or the like, and even in a case where the crotch region sags down due to weight of the absorber, a state is maintained in which these flaps are arranged on the waistline of the wearer.

Further, since the first elastic members are arranged in a stretched-out state in the longitudinal direction on the joining unit 51 between the dorsal side flap 20 and the diaper main body 10, the first elastic members can be pulled up towards the side flap side.

Specifically, a stretchable range of the first elastic members reaches the dorsal side flap 20, and the dorsal side flap 20 made from a material which is different from that of the diaper main body 10, is directly connected to the first elastic members. Therefore, when the dorsal side flap 20 is stretched out towards the ventral side, a linkage force from the dorsal side flap 30 to the first elastic members is increased.

For example, in a case where a stretchable range of the first elastic members is positioned at the inner side in the longitudinal direction with respect to the dorsal side flap 20, the dorsal side flap 20 and the first elastic members are not directly connected to each other. Therefore, when the dorsal side flap 20 is stretched out, force is disconnected between a stretch starting position of the first elastic members and the dorsal side flap 20, so that a linkage force from the dorsal side flap 20 to the first elastic members is decreased.

By overlapping a stretchable range of the first elastic members with the dorsal side flap 20, even in a case where the force for pulling down the crotch region of the diaper main body 10 is exerted due to weight of bodily fluid or the like, the stretching force of the first elastic members prevents the diaper main body from sagging down at the legs of the wearer, and the first elastic members can be arranged along the legs of the wearer. Therefore, the fitting ability can be maintained at the legs of the wearer, thereby achieving improvement in the feeling of comfort at the time of wearing while preventing side leakage.

Further, it is more desirable that a region in which the first elastic members of the dorsal side flap are arranged be contracted in a spread-out state by the first elastic members. For example, even in the condition that a stretchable region of the first elastic members is arranged at the dorsal side flap, in a case where the dorsal side flap is not contracted by the first elastic members, the force for bringing the dorsal side flap 20 in close contact with the skin is hardly exerted by the stretching force of the first elastic members, thereby possibly failing to increase the fitting ability at the legs of the wearer sufficiently.

However, in a case where the dorsal side flap is contracted by the first elastic members, the force for bringing the dorsal side flap 20 in close contact with the skin is exerted by the stretching force of the first elastic members, thereby achieving improvement in the fitting ability of both of the diaper main body 10 and the dorsal side flap 20 at the leg openings.

Further, when worn, because of the dorsal side flap 20, the force for pulling towards the outer side in the widthwise direction is exerted on the first elastic members, so that a position of the first elastic members can be maintained constantly in the direction of extending to the outside of the body. Therefore, displacement of the first elastic members due to movement of the legs and the buttocks of the wearer is reduced, so that the fitting ability with respect to the body can be maintained in a stable state.

Although the dorsal side flap 20 is configured independently of the diaper main body 10, the dorsal side flap 20 is formed in a sheet-like shape which continues across the entire diaper main body in the widthwise direction, so that the dorsal side flap 20 can increase tension strength in the widthwise direction as compared with a side flap which is divided in the widthwise direction. Therefore, the dorsal side flap 20 itself is not required to be configured from a material with a high base weight or material with high rigidity in order to increase strength of the dorsal side flap, so that the flexibility of the dorsal side flap 20 can be increased. By increasing the flexibility of the side flap, the sense of touch at the time of touching the skin is improved, thereby being able to prevent formation of hard wrinkles at the time of wearing.

In a case of forming the dorsal side flap 20 from a sheet which is continuous in the widthwise direction, it is desirable that, in the dorsal side flap, portions which are projecting outwardly from the diaper main body beyond the outer sides in the widthwise direction have a higher base weight than a portion which is overlapped with the diaper main body. A central portion between these portions projecting outwardly from the diaper main body beyond the outer sides in the widthwise direction is a portion with which the buttocks of the wearer come in contact, and a flexibility of this central portion can be increased. Further, by increasing the flexibility of the central portion of the dorsal side flap, a pocket can be easily formed between the dorsal side flap 20 and the topsheet 110.

Note that, a length in the longitudinal direction of a region in which a stretch range of the first elastic members and the dorsal side flap 20 are overlapped with each other is desirably within a range of 3 to 23% with respect to a length of the dorsal side flap 20 in the longitudinal direction. For example, in a case where such a region is longer than a length of 23%, the dorsal side flap 20 is possibly contracted too much by means of the first elastic members. If the dorsal side flap 20 is contracted too much in the longitudinal direction, the dorsal side flag 20 is deformed into a strand-shape at the time of wearing the diaper, so that the diaper cannot be held at the waistline, thereby leading to the possibility that the diaper is displaced.

On the other hand, in a case where the dorsal side flap is shorter than a length of 3%, a region in which the dorsal side flap 20 and the first elastic members are overlapped with each other is too narrow, so that the linkage between the dorsal side flap 20 and the first elastic members becomes weak. Therefore, the fitting ability at the leg openings cannot be increased enough.

Note that in the present embodiment, a length in the longitudinal direction of the region in which a stretch range of the first elastic members and the dorsal side flap 20 are overlapped with each other is equal to 18% with respect to a length of the dorsal side flap 20 in the longitudinal direction. Specifically, a length in the longitudinal direction of the dorsal side flap 20 is equal to 110 mm and a length of the region overlapped with the first elastic members is equal to 20 mm.

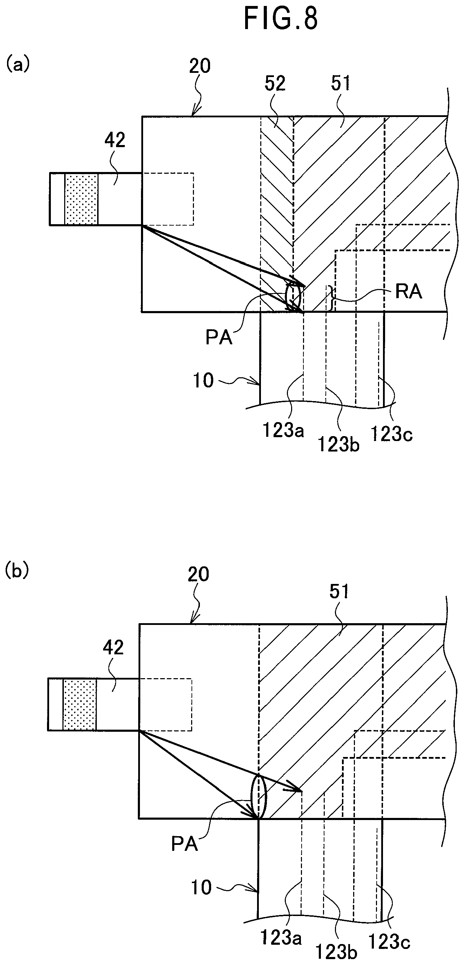

Further, at the outer sides in the widthwise direction with respect to the elastic members positioned at the outermost sides in the widthwise direction, non-joining units 52, 54 are provided, in which the side flap and the diaper main body are not joined. Fig. 8 is an enlarged view of part of the fixing unit of the dorsal side flap. Fig. 8 (a) shows the dorsal side flap according to the present embodiment and Fig. 8 (b) shows a dorsal side flap according to a modification which does not form part of the present invention. In the dorsal side flap according to the modification, the non-joining units 52, 54 are not provided at the outer sides in the widthwise direction with respect to the joining units.

The fixing members are arranged at the outer sides of the diaper main body in the widthwise direction, so that force transmitted from the fixing members to the diaper main body is exerted in the vicinity of the outer ends of the diaper main body in the widthwise direction. Therefore, force from the fixing members and the side flap is easily transmitted to the elastic members positioned at the outermost sides in the widthwise direction. However, if the joining units are provided at the outer sides in the widthwise direction with respect to the elastic members positioned at the outermost sides in the widthwise direction, the force from the fixing members is exerted on the joining units, thereby making it difficult to exert the force on the elastic members.

Specifically, the force exerted from the fixing members of the dorsal side flap 20 onto the diaper main body at the time of wearing the diaper is exerted on a portion PA positioned at the fixing member side of the joining unit. At this time, as shown in Fig. 8 (a), in a case where the non-joining units are provided, the part positioned at the fixing member side of the joining unit and the first elastic members are close to each other, so that the first elastic members can be interlocked with the dorsal side flap by effectively exerting the force from the fixing members onto the first elastic members.

On the other hand, as shown in Fig. 8 (b), in a case, being a reference example, where the non-joining units are not provided, the portion PA positioned at the fixing member side of the joining unit and the first elastic members are separated from each other, the force exerted from the fixing members is dispersed on the portion PA positioned at the fixing member side of the joining unit and on the first elastic members, thereby being not effectively exerted on the first elastic members.

Therefore, it is desirable that the non-joining units are provided at the outer sides in the widthwise direction with respect to the elastic members positioned at the outermost sides in the widthwise direction, respectively. By providing the non-joining units, the force from the side flap can be transmitted effectively to the elastic members, thereby being able to pull up the elastic members in association with pulling-up of the side flap.

(Manufacturing Method of Disposable Diaper)

Next, one example of a manufacturing method of the disposable diaper 1 is explained. As far as the other method that is not explained, the existing method can be used. Further, a manufacturing method explained hereinafter is one example and the disposable diaper 1 can also be manufactured by other methods. The manufacturing method of the disposable diaper 1 includes a folding step, a fixing-member attaching step, a first cutting step, a flap attaching step, and a second cutting step.

In the first folding step, both ends of a continuous side flap sheet, which configures the dorsal side flap and the ventral side flap, are folded back towards the central side in the widthwise direction. In this manner, the ends at both sides of the side flap sheet are provided with overlapped portions, respectively, in which the side flap sheet is folded to form multiple layers. The overlapped portions configure the free ends 20A, 20B of the dorsal side flap 20, for example.

In the fixing-member attaching step, the fixing members 41, 42 are attached to the free ends 20A, 20B of the dorsal side flap 20. In the first cutting step, the flap sheet is cut at predetermined intervals, along a crossing direction orthogonal to a conveyance direction. A cut piece is one united body of the dorsal side flap 20 and the ventral side flap 30.

In the flap attaching step, the cut piece is joined onto an absorbent body sheet configured by continuous absorbers. In the second cutting step, the absorbent body sheet is cut in the crossing direction. The cut piece is divided into a ventral side flap for one product and a dorsal side flap for another product subsequent to the aforementioned one product. Through the above steps, the disposable diaper 1 is completed.

As described above, by manufacturing the disposable diaper by joining the dorsal side flap and the ventral side flap to the absorbent body, no trims are wasted during manufacture of diapers. Since no extra materials are used, a product can be produced at lower cost while reducing burdens on the environment. Further, as described above, the first elastic members can fit onto the legs of the wearer and waste of extra material can be prevented while maintaining the fitting ability, like that of a pants-type diaper.

Out of the plurality of elastic members, the elastic members positioned at the outermost sides in the widthwise direction are arranged around the legs of the wearer, respectively. Each of the elastic members at the outermost sides in the widthwise direction is overlapped in an extended state with a joining unit configured to join the diaper main body and the side flap. Therefore, in association with pulling-up of the side flap, the elastic member positioned at each of the outermost sides in the widthwise direction is also pulled up together with the side flap.

Even in a case where the elastic member sags down due to weight of the bodily fluid or the like, the entire elastic member can be pulled up by the side flap towards the side flap side so as to arrange the elastic member along each of the legs of the wearer. Therefore, the fitting ability at the legs can be improved to thereby reduce a gap between the legs of the wearer and the disposable diaper, so that the side leakage of excretions can be controlled.

(Second Embodiment)

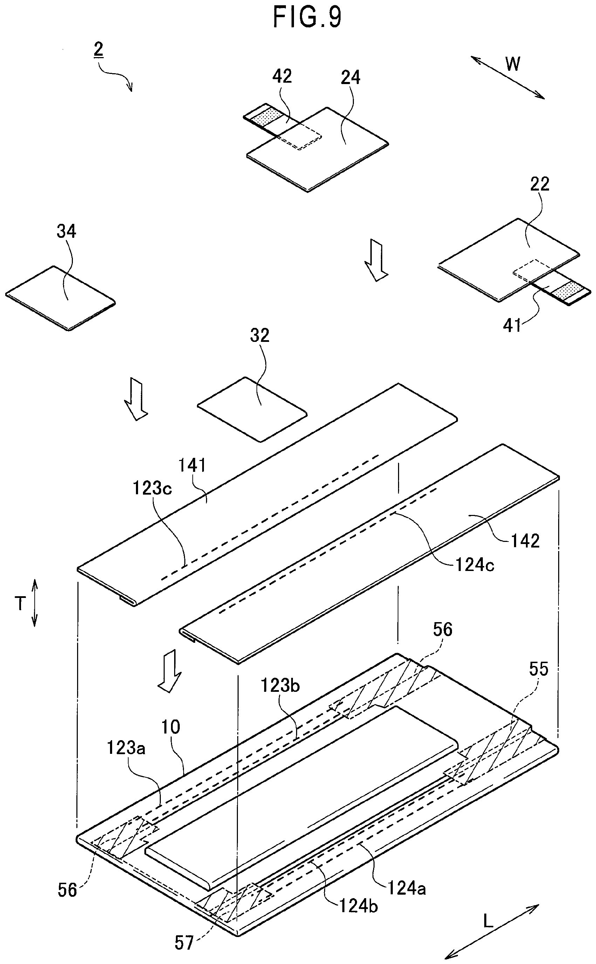

Next, a disposable diaper 2 according to a modification in the second embodiment is explained with reference to Fig. 9. In the following explanation of the embodiment, only the configuration that is different from the first embodiment is explained, and the same signs are used for the configuration that is the same as the first embodiment while omitting the explanation.

In the disposable diaper 2, side flaps are prepared as right and left individuals, and end regions 10A, 10B are not completely covered with the dorsal side flap and the ventral side flap. That is, the disposable diaper 2 has dorsal side flaps 22, 24. Further, the disposable diaper 2 has ventral side flaps 32, 34. The dorsal side flap 22 is joined to the diaper body 10 by means of a joining portion 55. The dorsal side flap 24 is joined to the diaper main body 10 by means of a joining unit 56. The ventral side flap 32 is joined to the diaper main body 10 by means of a joining unit 57. The ventral side flap 34 is joined to the diaper main body 10 by means of a joining unit 58. The disposable diaper 2 also attains the same effect as the disposable diaper 1.

(Other Embodiment)

So far, the present invention is disclosed through the above embodiments. However, it should not be interpreted that the statements and drawings configuring a part of the present disclosure limit the present invention. From this disclosure, a variety of alternate embodiments, examples, and applicable techniques will become apparent to one skilled in the art.

In the present embodiments, described is a disposable diaper 1 in which the dorsal side flap 20 and the ventral side flap 30 are mounted on the diaper main body 10. However, an embodiment of the disposable diaper is not limited to the present embodiments. For example, the disposable diaper 1 may be an open-type which is formed by uniting a waistline region corresponding to the waistline of the wearer and a crotch region corresponding to the crotch portion of the wearer, and by hollowing out openings for the legs of the wearer. Alternatively, the disposable diaper 1 may not include the ventral side flap. The disposable diaper 1 may be for any of infants, toddlers, and adults.

In the present embodiments, the first elastic members are arranged in a stretched-out state in the joining unit between the dorsal side flap and the diaper main body. However, the first elastic members may be arranged at the inner side in the longitudinal direction with respect to the joining unit between the ventral side flap and the diaper main body or may be arranged in an unstretched state at the joining portion between the ventral side flap and the diaper main body.

The disposable diaper according to the present embodiments is provided with the non-joining units at the outer sides in the widthwise direction with respect to the joining unit.

In the disposable diaper according to the present embodiments, the dorsal side flap is formed so that a base weight is higher at only both ends in the widthwise direction as compared with the central portion. However, the disposable diaper may be configured so that a base weight is equal across the entire region of the dorsal side flap or is higher at the central portion as compared with both ends in the widthwise direction.

Note that although the present embodiments is configured so that the plurality of first elastic members 123a, 123b, 124a, 124b are overlapped with the joining unit 51 in the thickness direction, there is no limitation to this configuration. Specifically, it is sufficient that, out of the plurality of elastic members, the elastic members (the first elastic members 123a, 124a in the present embodiments) positioned at the outermost sides in the widthwise direction be arranged so as to be overlapped with the joining unit 51. Thus, only the first elastic members 123a, 123a may be overlapped with the joining unit 51 or three or more elastic members including the first elastic members 123a, 124a may be arranged so as to be overlapped with the joining unit 51.

As described above, needless to say, the present invention includes various embodiments and the like not described here. Therefore, the technical range of the present invention is to be defined only by the inventive specific matter according to the satisfactory claims from the above description.

[Embodiment]

By citing embodiments and comparative examples, bending stiffness and bending recovery, which are indicative of a bending property, of the fixing member and the side flap were measured to evaluate each material in terms of adequacy for the fixing member and the side flap. Note that this does not limit the present invention.

Using materials according to the first and second embodiments, and materials according to the first and the second comparative examples, bending stiffness and bending recovery indicative of a bending property were measured. At the same time, each material was evaluated in terms of adequacy for the fixing member. The adequacy for the fixing member was evaluated based on stiffness and resistance to displacement.

Stiffness causing no feeling of discomfort at the time of contact with the skin of the wearer was evaluated as "good", whereas stiffness possibly causing feeling of discomfort was evaluated as "bad". The material which was used as the fixing member and caused no displacement from the dorsal side flap or the ventral side flap was evaluated as "good", whereas the material which caused displacement was evaluated as "bad".

Further, using the materials according to the third and fourth embodiments and the materials according to the third and fourth comparative examples, bending stiffness and bending recovery indicative of a bending property were measured. At the same time, each material was evaluated in terms of adequacy for the side flap. The adequacy for the side flap was evaluated based on stiffness and resistance to tear.

Stiffness causing no feeling of discomfort at the time of contact with the skin of the wearer was evaluated as "good", whereas stiffness possibly causing feeling of discomfort was evaluated as "bad". The material which was used as the dorsal side flap and was not torn was evaluated as "good", whereas the material which was torn was evaluated as "bad".

(Measuring Method of Bending Stiffness and Bending Recovery)

The bending stiffness and the bending recovery were measured by a KES method. KES FB-2 was used as a measuring instrument. First, for each material, a sample with a height of 100 mm and a width of 30 mm is obtained. The sample is obtained so that the height is oriented along a conveyance direction in a manufacturing step of a material and the width is oriented along a crossing direction orthogonal to the conveyance direction. After the measuring instrument is set to zero, the sample is set in a manner to be bent at right angles to a longitudinal direction, and the measurement is started. Values of B-2HB displayed on a screen of the measuring instrument are recorded. Subsequently, the sample is set in a manner to be bent at right angles to a lateral direction, and the measurement is started. Values of B-2HB displayed on the screen of the measuring instrument are recorded.

Bending stiffness is calculated by an equation indicated in Equation 1.

[Equation 1]

$$\text{Bending Stiffness} = \frac{\text{Direction MD Value B} + \text{Direction CD Value B}}{2} \ (\times 10^{-4} \ \text{Nm/m})$$

Bending recovery is calculated by an equation indicated in Equation 2.

[Equation 2]

$$\text{Bending Recovery} = \frac{\text{Direction MD Value 2HB} + \text{Direction CD Value 2HB}}{2} \ (\times 10^{-2} \ \text{Nm/m})$$

(Measurement Result)

Measurement results are shown in Table 1. Note that a measured value is rounded to two decimal places and is shown with two decimal places.

[Table 1]

| Fixing Member | 1st Embodiment | 2nd Embodiment | 1st Comparative Example | 2nd Comparative Example |
|---|---|---|---|---|
| Stiffness | Good | Good | Bad | Good |
| Resistance to Displacement | Good | Good | Good | Bad |
| Bending Stiffness | 0.68 | 0.56 | 0.90 | 0.07 |
| Direction MD Value B | 0.82 | 0.62 | 1.07 | 0.09 |
| Direction CD Value B | 0.53 | 0.50 | 0.73 | 0.05 |
| Bending Recovery | 0.74 | 0.52 | 0.95 | 0.08 |
| Direction MD Value 2HB | 1.03 | 0.66 | 1.33 | 0.10 |
| Direction CD Value 2HB | 0.44 | 0.38 | 0.57 | 0.05 |
| Side Flap | 3rd Embodiment | 4th Embodiment | 3rd Comparative Example | 4th Comparative Example |
| Stiffness | Good | Good | Bad | Good |
| Resistance to Tear | Good | Good | Good | Bad |
| Bending Stiffness | 0.07 | 0.07 | 0.56 | 0.01 |

(continued)

| Side Flap | 3rd Embodiment | 4th Embodiment | 3rd Comparative Example | 4th Comparative Example |
|---|---|---|---|---|
| Direction MD Value B | 0.09 | 0.10 | 0.62 | 0.02 |
| Direction CD Value B | 0.05 | 0.05 | 0.50 | 0.00 |
| Bending Recovery | 0.08 | 0.07 | 0.52 | 0.02 |
| Direction MD Value 2HB | 0.10 | 0.09 | 0.66 | 0.03 |
| Direction CD Value 2HB | 0.05 | 0.05 | 0.38 | 0.01 |

The material according to the first comparative example is obtained by performing a laminating process onto both surfaces of polypropylene spunbonded nonwoven fabric having a basis weight of 80 g/ cm$^2$. This material has the highest bending stiffness and bending recovery among the four materials. Since the material is stiff, it was evaluated as inadequate for the fixing member.

The material according to the second comparative example is polypropylene spunbonded nonwoven fabric having a basis weight of 30 g/ cm$^2$. This material has the lowest bending stiffness and bending recovery among the four materials. Since the material is soft enough to cause displacement, it was evaluated as inadequate for the fixing member.

The material according to the first embodiment is a polypropylene spunbonded nonwoven fabric having a basis weight of 80 g/ cm$^2$. In terms of stiffness and resistance to displacement, this material was evaluated as adequate for the fixing member.

The material according to the second embodiment is a polypropylene spunbonded nonwoven fabric having a basis weight of 60 g/ cm$^2$. In terms of stiffness and resistance to displacement, this material was evaluated as adequate for the fixing member.

According to the above results, the bending stiffness of the fixing member is desired to fall within a range from $0.56 \times 10^{-4}$ Nm/m to $0.68 \times 10^{-4}$ Nm/m, whereas the bending recovery of the fixing member is desired to fall within a range from $0.52 \times 10^{-2}$ Nm/m to $0.74 \times 10^{-2}$ Nm/m.

The material according to the third comparative example is a polypropylene spunbonded nonwoven fabric having a basis weight of 60 g/ cm$^2$. This material has the highest bending stiffness and bending recovery among the four materials. Since the material is stiff, it was evaluated as inadequate for the dorsal side flap.

The material according to the fourth comparative example is an SMS nonwoven cloth having a basis weight of 15 g/ cm$^2$. This material has the lowest bending stiffness and bending recovery among the four materials. Since the material is soft enough to be torn, it was evaluated as inadequate for the dorsal side flap

The material according to the third embodiment is a polypropylene spunbonded nonwoven fabric having a basis weight of 30 g/ cm$^2$. In terms of stiffness and resistance to tear, this material was evaluated as adequate for the dorsal side flap.

The material according to the fourth embodiment is an SMS nonwoven cloth having a basis weight of 15 g/ cm$^2$. In terms of stiffness and resistance to displacement, this material was evaluated as adequate for the dorsal side flap.

According to the above results, the bending stiffness of the dorsal side flap is desired to be equal to $0.07 \times 10^{-4}$ Nm/m, whereas the bending recovery of the dorsal side flap is desired to fall within a range from $0.07 \times 10^{-2}$ Nm/m to $0.08 \times 10^{-2}$ Nm/m.

In a case where the bending stiffness of the side flap is less than $0.07 \times 10^{-4}$ Nm/m or the bending recovery is less than $0.07 \times 10^{-2}$ Nm/m, the strength of the side flap is not enough, and there is a possibility that the diaper is broken when worn or that the side flap is turned up when worn.

On the other hand, in a case where the bending stiffness of the side flap is larger than $0.07 \times 10^{-4}$ Nm/m or the bending recovery is larger than $0.08 \times 10^{-2}$ Nm/m, there is a possibility that wrinkles are formed when the side flap is stretched out because of the elastic members, thereby increasing a stimulation to the skin.

[Industrial Applicability]

According to the characteristic provided by some embodiments of the present invention, it is possible to provide a disposable diaper which is capable of improving the fitting ability at legs of the wearer to thereby control occurrence of side leakage by arranging elastic members configuring leg gathers running around the legs of a wearer.

[Reference Signs List]

1...Disposable diaper
10...Diaper main body
10A,10B...End region
13,14...Leg gathers
15,16...Leakage-preventing walls
20...Dorsal side flap
20A,20B...Free end
30...Ventral side flap
41,42...Fixing member
51,53...Joining portion
110...Topsheet
120... Exterior sheet
121...Back nonwoven fabric
122...Back film
123a,123b,124a,124b...First elastic member
123c,124c...Second elastic member
130...Absorber
131...Absorbent core
132...Absorbent sheet
141,142... Sidesheet

**Claims**

1. A disposable diaper (1) comprising:

   a diaper main body (10) including:

   a liquid-permeable topsheet (110);
   a liquid-impermeable backsheet (120);
   an absorber (130) provided between the topsheet and the backsheet; and
   a plurality of elastic members (123a, 123b, 124a, 124b,123c, 124c) arranged in an extended state along a longitudinal direction (L) of the absorber, the elastic members including first elastic members (123a, 123b, 124a, 124b) at outer sides in a widthwise direction with respect to the absorber for configuring leg gathers (13) formed around the legs of a wearer, and side elastic members (123c, 124c) for configuring leakage-preventing walls (15);
   a dorsal side flap (20, 22, 24) arranged at one outer end of the diaper main body in the longitudinal direction at a dorsal side of a wearer in use and projecting outwardly beyond the outer sides in the widthwise direction of the diaper main body;
   a ventral side flap (30, 32, 34) arranged at the other outer end of the diaper main body in the longitudinal direction at a ventral side of the wearer in use and extending outwardly beyond the outer sides in the widthwise direction of the diaper main body; and
   a joining unit (51, 55, 56) being a region where the dorsal side flap and the diaper main body are joined to each other, wherein
   dorsal ends (123ar, 124ar,) of the first elastic members (123a, 124a) positioned at outermost sides in the widthwise direction out of the plurality of elastic members are overlapped, in an extended state in the longitudinal direction, with the joining unit in a thickness direction (T) of the disposable diaper, the thickness direction being perpendicular to the longitudinal and widthwise directions of the disposable diaper;
   a non-joining unit (52) in which the dorsal side flap and the diaper main body are not joined together is provided at each outer side in the widthwise direction, with respect to the first elastic members (123a, 124a) positioned at the outermost sides in the widthwise direction, and with respect to the joining unit;
   the dorsal side flap is provided with a fixing member (41, 42) which is joined to both outer ends of the dorsal side flap in the widthwise direction and is configured to fix the dorsal side flap in an attachable and detachable manner in use to the diaper main body or the ventral side flap arranged at the ventral side of the wearer;

the disposable diaper includes a pair of sidesheets (141, 142) which cover both ends of the absorber in the widthwise direction, and are arranged separately from each other in the widthwise direction;

the ends of the sidesheets in the longitudinal direction are arranged between the topsheet and the dorsal side flap and are joined to each of the topsheet and the dorsal side flap in a region which is overlapped with the absorber in the thickness direction;

a side elastic member (123c, 124c), configured to be stretched in the longitudinal direction, is provided at an inner end of each sidesheet in the widthwise direction for configuring a leakage-preventing wall (15) at a crotch portion of a wearer; and

in the joining unit (51), the dorsal side flap (20), the sidesheets (141, 142) and the back sheet (120) are adapted to be contracted in the longitudinal direction by means of the first elastic members (123a,124a).

2. The disposable diaper according to claim 1, wherein:

the dorsal side flap (20, 22, 24) is overlapped with one end of the diaper main body in the longitudinal direction; and the ventral side flap (30, 32, 34) is overlapped with the other end of the diaper main body in the longitudinal direction.

**Patentansprüche**

1. Einwegwindel (1), die Folgendes umfasst:

einen Windelhauptkörper (10), der Folgendes einschließt:

eine flüssigkeitsdurchlässige obere Lage (110);
eine flüssigkeitsundurchlässige hintere Lage (120);
einen Absorber (130), der zwischen der oberen Lage und der hinteren Lage bereitgestellt ist; und
eine Vielzahl von elastischen Elementen (123a, 123b, 124a, 124b, 123c, 124c), die in einem ausgedehnten Zustand entlang einer Längsrichtung (L) des Absorbers angeordnet sind, wobei die elastischen Elemente Folgendes einschließen: erste elastische Elemente (123a, 123b, 124a, 124b) an äußeren Seiten in einer Breitenrichtung in Bezug auf den Absorber für die Konfiguration von Beinraffungen (13), die um die Beine eines Trägers ausgebildet sind, und seitliche elastische Elemente (123c, 124c) für die Konfiguration von Auslauf-verhindernden Wänden (15);
eine Dorsalseitenklappe (20, 22, 24), die an einem äußeren Ende des Windelhauptkörpers in der Längsrichtung an einer dorsalen Seite eines Trägers bei Verwendung angeordnet ist und nach außen über die äußeren Seiten in der Breitenrichtung des Windelhauptkörpers hinaus herausragt;
eine Ventralseitenklappe (30, 32, 34), die an dem anderen äußeren Ende des Windelhauptkörpers in der Längsrichtung an einer ventralen Seite des Trägers bei Verwendung angeordnet ist und sich nach außen über die äußeren Seiten in der Breitenrichtung des Windelhauptkörpers hinaus erstreckt; und
eine Verbindungseinheit (51, 55, 56), die ein Bereich ist, wo die Dorsalseitenklappe und der Windelhauptkörper miteinander verbunden sind, wobei
dorsale Enden (123ar, 124ar) der ersten elastischen Elemente (123a, 124a), die an äußersten Seiten in der Breitenrichtung, von der Vielzahl der elastischen Elemente, positioniert sind, in einem ausgedehnten Zustand in der Längsrichtung mit der Verbindungseinheit in einer Dickenrichtung (T) der Einwegwindel überlappen, wobei die Dickenrichtung senkrecht zu der Längs- und Breitenrichtung der Einwegwindel verläuft;
eine Nicht-Verbindungseinheit (52), in der die Dorsalseitenklappe und der Windelhauptkörper nicht miteinander verbunden sind, an jeder äußeren Seite in der Breitenrichtung in Bezug auf die ersten elastischen Elemente (123a, 124a), die an den äußersten Seiten in der Breitenrichtung positioniert sind, und in Bezug auf die Verbindungseinheit bereitgestellt ist;
die Dorsalseitenklappe mit einem fixierenden Element (41, 42) bereitgestellt ist, das mit beiden äußeren Enden der Dorsalseitenklappe in der Breitenrichtung verbunden ist und zur Fixierung der Dorsalseitenklappe in einer anfügbaren und lösbaren Weise bei Verwendung an den Windelhauptkörper oder die Ventralseitenklappe, die an der ventralen Seite des Trägers angeordnet ist, konfiguriert ist;
die Einwegwindel ein Paar von Seitenlagen (141, 142) einschließt, die beide Enden des Absorbers in der Breitenrichtung abdecken und getrennt voneinander in der Breitenrichtung angeordnet sind;
die Enden der Seitenlagen in der Längsrichtung zwischen der oberen Lage und der Dorsalseitenklappe angeordnet sind und mit jeder von der oberen Lage und der Dorsalseitenklappe in einem Bereich verbunden sind, der mit dem Absorber in der Dickenrichtung überlappt;

ein seitliches elastisches Element (123c, 124c), das zur Dehnung in der Längsrichtung konfiguriert ist, an einem inneren Ende von jeder Seitenlage in der Breitenrichtung bereitgestellt ist, um eine Auslauf-verhindernde Wand (15) an einem Schrittabschnitt eines Trägers zu konfigurieren; und

in der Verbindungseinheit (51) die Dorsalseitenklappe (20), die Seitenlagen (141, 142) und die hintere Lage (120) geeignet sind, um in der Längsrichtung mithilfe der ersten elastischen Elemente (123a, 124a) zusammengezogen zu werden.

2. Einwegwindel nach Anspruch 1, wobei:

die Dorsalseitenklappe (20, 22, 24) mit einem Ende des Windelhauptkörpers in der Längsrichtung überlappt; und die Ventralseitenklappe (30, 32, 34) mit dem anderen Ende des Windelhauptkörpers in der Längsrichtung überlappt.

**Revendications**

1. Couche-culotte jetable (1) comprenant :

un corps principal de couche-culotte (10) incluant :

une feuille supérieure perméable aux liquides (110) ;
une feuille de support imperméable aux liquides (120) ;
un absorbant (130) disposé entre la feuille supérieure et la feuille de support ; et
une pluralité d'éléments élastiques (123a, 123b, 124a, 124b, 123c, 124c) disposés, en extension, le long d'une direction de longueur (L) de l'absorbant, les éléments élastiques incluant des premiers éléments élastiques (123a, 123b, 124a, 124b) disposés sur les côtés externes dans une direction de largeur par rapport à l'absorbant pour constituer des fronces jambières (13) formées autour des jambes d'un porteur et des éléments élastiques latéraux (123c, 124c) pour constituer des parois empêchant les fuites (15) ;
un volet latéral dorsal (20, 22, 24) disposé à une extrémité externe du corps principal de la couche-culotte dans la direction de longueur sur un côté dorsal d'un porteur à l'utilisation et se projetant vers l'extérieur au-delà des côtés externes dans la direction de largeur du corps principal de la couche-culotte ;
un volet latéral ventral (30, 32, 34) disposé à l'autre extrémité externe du corps principal de la couche-culotte dans la direction de longueur sur un côté ventral du porteur à l'utilisation et projetant vers l'extérieur au-delà des côtés externes dans la direction de largeur du corps principal de la couche-culotte ; et
une unité de jonction (51, 55, 56) correspondant à une région où le volet latéral dorsal et le corps principal de la couche-culotte sont joints l'un à l'autre, où
les extrémités dorsales (123ar, 124ar) des premiers éléments élastiques (123a, 124a) de la pluralité d'éléments élastiques qui sont disposés sur les côtés les plus externes dans la direction de largeur sont, en extension, en chevauchement dans la direction de longueur avec l'unité de jonction dans une direction d'épaisseur (T) de la couche-culotte jetable, la direction d'épaisseur étant perpendiculaire aux directions de longueur et de largeur de la couche-culotte jetable ;
une unité ne définissant pas une jonction (52) dans laquelle le volet latéral dorsal et le corps principal de la couche-culotte ne sont pas joints ensemble est fournie sur chaque côté externe dans la direction de largeur par rapport aux premiers éléments élastiques (123a, 124a) disposés sur les côtés les plus externes dans la direction de largeur et par rapport à l'unité de jonction ;
le volet latéral dorsal est doté d'un élément de fixation (41, 42) qui est joint aux deux extrémités externes du volet latéral dorsal dans la direction de largeur et qui est conçu pour fixer le volet latéral dorsal d'une manière rattachable et détachable à l'utilisation ;
la couche-culotte jetable inclut une paire de feuilles latérales (141, 142) qui couvrent les deux extrémités de l'absorbant dans la direction de largeur et qui sont disposées séparément l'une de l'autre dans la direction de largeur ;
dans la direction de longueur, les extrémités des feuilles latérales sont disposées entre la feuille supérieure et le volet latéral dorsal et sont jointes à la fois à la feuille supérieure et au volet latéral dorsal dans une région qui est en chevauchement avec l'absorbant dans la direction d'épaisseur ;
un élément élastique latéral (123c, 124c), conçu pour être étiré dans la direction de longueur, est fourni à une extrémité interne de chaque feuille latérale dans la direction de largeur pour constituer une paroi empêchant les fuites (15) au niveau d'une région d'entrejambe d'un porteur ; et

dans l'unité de jonction (51), le volet latéral dorsal (20), les feuilles latérales (141, 142) et la feuille de support (120) sont conçus pour être contractés dans la direction de longueur au moyen des premiers éléments élastiques (123a, 124a).

2. Couche-culotte jetable selon la revendication 1, où :

le volet latéral dorsal (20, 22, 24) est en chevauchement avec une extrémité du corps principal de la couche-culotte dans la direction de longueur ; et

le volet latéral ventral (30, 32, 34) est en chevauchement avec l'autre extrémité du corps principal de la couche-culotte dans la direction de longueur.

# FIG.1

FIG.2

# FIG.3

# FIG.4

# FIG.5

# FIG.6

# FIG.7

# FIG.8

(a)

20

52    51

42

RA

PA

10

123a    123b    123c

(b)

20

51

42

PA

10

123a    123b    123c

# FIG.9

**EP 2 752 177 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000513636 A
- WO 2011040046 A
- WO 2011040045 A